# DEMANDE DE BREVET EUROPEEN

(11) **EP 1 649 896 A2**
(43) Date de publication de la demande: **26.04.2006**
(21) Numéro de dépôt: 05292153.3
(22) Date de dépôt: 13.10.2005
(51) Int. Cl.: A61Q 5/06, A61K 8/40

(54) **Composition de coloration comprenant un mélange de pigment particulier et au moins un monomére électrophile**

(30) Priorité: 13.10.2004 FR 0410802
(71) Demandeur: L'OREAL, 75008 Paris (FR)
(72) Inventeur: Brun, Gaëlle, 75015 Paris (FR)
(74) Mandataire: Fevrier, Murielle Françoise E.

(57) **Abrégé**

La présente invention qui a pour objet une composition de coloration comprenant, dans un milieu de coloration approprié, au moins un monomère électrophile, au moins un pigment coloré et au moins un pigment d'un indice de réfraction supérieur au pigment coloré.

La composition conforme à la présente invention permet d'améliorer la visibilité de la coloration sur une matière kératinique foncée.

## Description

La présente invention a pour objet une composition de coloration des matières kératiniques, en particulier des fibres kératiniques telles que les cheveux, comprenant au moins un mélange de pigments particuliers et au moins un monomère électrophile.

Dans le domaine de la coloration des fibres kératiniques, il est déjà connu de colorer des fibres kératiniques par différentes techniques à partir de colorants directs ou de pigments pour des colorations non permanente ou de précurseurs de colorant pour des colorations permanentes.

La coloration non permanente ou coloration directe consiste à teindre les fibres kératiniques avec des compositions tinctoriales contenant des colorants directs. Ces colorants sont des molécules colorées et colorantes ayant une affinité pour les fibres kératiniques. Ils sont appliqués sur les fibres kératiniques pendant un temps nécessaire à l'obtention de la coloration désirée, puis rincés.

Les colorants classiques qui sont utilisés sont en particulier des colorants du type nitré benzénique, anthraquinonique, nitropyridinique, azoïque, xanthénique, acridinique, azinique, triarylméthane ou des colorants naturels.

Certains de ces colorants peuvent être utilisés dans des conditions éclaircissantes ce qui permet d'obtenir des colorations visibles sur des cheveux foncés.

Il est aussi connu de teindre les fibres kératiniques de façon permanente par la coloration d'oxydation. Cette technique de coloration consiste à appliquer sur les fibres kératiniques une composition contenant des précurseurs de colorant tels que des bases d'oxydation et des coupleurs. Ces précurseurs sous l'action d'un agent oxydant vont former dans le cheveu une ou plusieurs espèces colorées.

La variété des molécules mises en jeu au niveau des bases d'oxydation et des coupleurs, permet l'obtention d'une riche palette de couleurs et les colorations qui en résultent sont permanentes, puissantes, et résistantes aux agents extérieurs, notamment à la lumière, aux intempéries, aux lavages, à la transpiration et aux frottements.

Pour être visible sur cheveux foncés, ces deux techniques de coloration nécessitent une décoloration préalable ou simultanée des fibres kératiniques. Cette étape de décoloration mise en oeuvre avec un agent oxydant tel que le peroxyde d'hydrogène ou de persels entraîne une dégradation non négligeable des fibres kératiniques ce qui altère leurs propriétés cosmétiques. Les cheveux ont alors tendance à devenir rêches, plus difficilement démêlables et plus fragiles.

Une autre méthode de coloration consiste à utiliser des pigments. En effet, l'utilisation de pigment à la surface des fibres kératiniques permet en général d'obtenir des colorations visibles sur cheveux foncés puisque le pigment en surface masque la couleur naturelle de la fibre. L'utilisation de pigment pour colorer des fibres kératiniques est par exemple décrite dans la demande de brevet FR 2 741 530, qui préconise l'utilisation pour la coloration des fibres kératiniques d'une composition comprenant au moins une dispersion de particules de polymère filmogène comportant au moins une fonction acide et au moins un pigment dispersé dans la phase continue de ladite dispersion.

Les colorations obtenues par ce mode de coloration présentent l'inconvénient d'avoir une faible résistance aux shampooings.

Par ailleurs, il est connu de la demande de brevet FR 2 833 489 des compositions de traitement des cheveux à partir de compositions comprenant des monomères électrophiles. Une telle composition permet d'obtenir des cheveux parfaitement gainés et non gras.

Le but de la présente invention est de fournir de nouvelles compositions pour la coloration des matières kératiniques, et en particulier des fibres kératiniques telles que les cheveux, qui permettent d'obtenir des colorations visibles sur cheveux foncés sans qu'il soit nécessaire d'éclaircir ou de décolorer les fibres et qui présentent une bonne résistance aux shampooings.

Ce but est atteint avec la présente invention qui a pour objet une composition de coloration comprenant, dans un milieu de coloration approprié, au moins un monomère électrophile, au moins un pigment coloré et au moins un pigment d'un indice de réfraction supérieur au pigment coloré.

La composition conforme à la présente invention permet d'améliorer la visibilité de la coloration sur une matière kératinique foncée. En particulier, dans le cas des fibres kératiniques foncées, on obtient une coloration très visible sans qu'il soit nécessaire d'éclaircir ou de décolorer les fibres kératiniques et par conséquent sans dégradation physique des fibres kératiniques. De plus, l'association de pigments de la composition de l'invention permet d'obtenir des compositions plus couvrantes ce qui permet de mieux masquer les cheveux blancs.

De plus, cette coloration présente une bonne résistance aux diverses agressions que peuvent subir les cheveux telles que les shampooings, les frottements, la lumière, les intempéries, la sueur et les déformations permanentes. Ces propriétés sont particulièrement remarquables en ce qui concerne la résistance de la coloration vis à vis des shampooings.

La présente invention a aussi pour objet un procédé de coloration des matières kératiniques qui comprend l'application sur les matières kératiniques de la composition de l'invention ainsi que l'utilisation de la composition de l'invention pour la coloration des matières kératiniques, en particulier les fibres kératiniques tels que les cheveux.

Un autre objet de l'invention concerne un kit comprenant d'une part un mélange de pigments tel que défini précédemment et d'autre part un monomère électrophile.

Dans le cadre de l'invention, les pigments présentent une solubilité dans l'eau inférieure à 0,01 % à 20 °C, de préférence inférieure à 0,0001 %, et présentant une absorption entre 350 et 700 nm, de préférence une absorption avec un maximum.

Les pigments utiles dans la présente invention peuvent se présenter sous forme de poudre ou de pâte pigmentaire.

Le pigment coloré utile dans la composition de l'invention peut-être un pigment organique. Par pigment organique on entend tout pigment qui répond à la définition de l'encyclopédie Ullmann dans le chapitre pigment organique. Le pigment organique peut notamment être choisi parmi les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

Le ou les pigments organiques peuvent être choisis par exemple parmi parmi le carmin, le noir de carbone, le noir d'aniline, le jaune azo, la quinacridone, le bleu de phtalocyanine, le rouge sorgho, les pigments bleus codifiés dans le Color Index sous les références CI 42090, 69800, 69825, 73000, 74100, 74160, les pigments jaunes codifiés dans le Color Index sous les références CI 11680, 11710, 15985, 19140, 20040, 21100, 21108, 47000, 47005, les pigments verts codifiés dans le Color Index sous les références CI 61565, 61570, 74260, les pigments oranges codifiés dans le Color Index sous les réfénces CI 11725, 15510, 45370, 71105, les pigments rouges codifiés dans le Color Index sous les références CI 12085, 12120, 12370, 12420, 12490, 14700, 15525, 15580, 15620, 15630, 15800, 15850, 15865, 15880, 17200, 26100, 45380, 45410, 58000, 73360, 73915, 75470, les pigments obtenus par polymérisation oxydante de dérivés indoliques, phénoliques tels qu'ils sont décrits dans le brevet FR 2 679 771.

Ces pigments peuvent aussi être sous forme de pigments composites tels qu'ils sont décrits dans le brevet EP 1 184 426. Ce pigment composite peut être composé notamment de particules comportant un noyau inorganique, au moins un liant assurant la fixation des pigments organiques sur le noyau, et au moins un pigment organique recouvrant au moins partiellement le noyau

A titre d'exemple, on peut aussi citer les pâtes pigmentaires de pigment organique telles que les produits vendus par la société HOECHST sous le nom :
- JAUNE COSMENYL IOG : Pigment YELLOW 3 (Cl 11710)
- JAUNE COSMENYL G : Pigment YELLOW 1 (CI 11680)
- ORANGE COSMENYL GR : Pigment ORANGE 43 (Cl 71105)
- ROUGE COSMENYL R" : Pigment RED 4 (CI 12085)
- CARMIN COSMENYL FB : Pigment RED 5 (Cl 12490)
- VIOLET COSMENYL RL : Pigment VIOLET 23 (Cl 51319)
- BLEU COSMENYL A2R : Pigment BLUE 15.1 (CI 74160)
- VERT COSMENYL GG : Pigment GREEN 7 (CI 74260)
- NOIR COSMENYL R Pigment BLACK 7 (CI 77266)

Le pigment coloré peut aussi être une laque. Par laque, on entend les colorants adsorbés sur des particules insolubles, l'ensemble ainsi obtenu restant insoluble lors de l'utilisation. Les substrats inorganiques sur lesquels sont adsorbés les colorants sont par exemple l'alumine, la silice, le borosilicate de calcium et de sodium ou le borosilicate de calcium et d'aluminium, et l'aluminium. Parmi les colorants organiques, on peut citer le carmin de cochenille.

A titre d'exemples de laques, on peut citer les produits connus sous les dénominations suivantes : D & C Red 21 (CI 45 380), D & C Orange 5 (Cl 45 370), D & C Red 27 (CI 45 410), D & C Orange 10 (CI 45 425), D & C Red 3 (CI 45 430), D & C Red 7 (CI 15 850:1), D & C Red 4 (CI 15 510), D & C Red 33 (Cl 17 200), D & C Yellow 5 (CI 19 140), D & C Yellow 6 (CI 15 985), D & C Green (CI 61 570), D & C Yellow 1 O (CI 77 002), D & C Green 3 (CI 42 053), D & C Blue 1 (Cl 42 090).

Le pigment coloré peut aussi être un pigment à effets spéciaux. Par pigments à effets spéciaux, on entend les pigments qui créent d'une manière générale une apparence colorée (caractérisée par une certaine nuance, une certaine vivacité et une certaine clarté) non uniforme et changeante en fonction des conditions d'observation (lumière, température, angles d'observation...). Ils s'opposent par-là même aux pigments blancs ou colorés qui procurent une teinte uniforme opaque, semi-transparente ou transparente classique.

Il existe deux types de pigments à effets spéciaux ceux à faible indice de réfraction tels qu les pigments fluorescents, photochromes ou thermochromes et ceux à plus fort indice de réfraction tels que les nacres ou les paillettes.

A titre de pigments à effets spéciaux, on peut citer les pigments nacrés tels que les pigments nacrés blancs tels que le mica titane, ou mica-oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les pigments à effets spéciaux comprennent aussi les pigments fluorescents, que ce soit les substances fluorescentes à la lumière du jour ou qui produisent une fluorescence ultraviolette, les pigments phosphorescents, les pigments photochromiques, les pigments thermochromiques.

La composition de l'invention contient aussi un ou plusieurs pigments ayant un indice de réfraction supérieur au pigment coloré.

Le pigment ou les pigments ayant un indice de réfraction plus élevé que le ou les pigments colorés peuvent être des pigments minéraux. Par pigment minéral on entend tout pigment qui répond à la définition de l'encyclopédie Ullmann dans le chapitre pigment inorganique. On peut citer, parmi les pigments minéraux utiles dans la présente invention les oxydes de zirconium ou de cérium, ainsi que les oxydes de fer ou de chrome, le violet de manganèse, le bleu outremer, l'hydrate de chrome et le bleu ferrique, le dioxyde de titane. Les pigments minéraux suivants peuvent aussi être utilisés : Ta₂O₅, Ti₃O₅, Ti₂O₃, TiO, ZrO₂ en mélange avec TiO₂, ZrO₂, Nb₂O₅, CeO₂, ZnS.

Le pigment ou les pigments ayant un indice de réfraction plus élevé que le ou les pigments colorés peuvent aussi être des pigments nacrés tels que des pigments nacrés blancs par exemple le mica recouvert de titane, ou d'oxychlorure de bismuth, des pigments nacrés colorés tels que le mica recouvert de titane et d'oxydes de fer, le mica recouvert de titane et notamment de bleu ferrique ou d'oxyde de chrome, le mica recouvert de titane et d'un pigment organique tel que défini précédemment ainsi que les pigments nacrés à base d'oxychlorure de bismuth. A titre d'exemple, on peut citer les pigments Cellini commercialisée par Engelhard (Mica-TiO2-laque), Prestige commercialisée par Eckart (Mica-TiO2), Colorona commercialisée par Merck (Mica-TiO2-Fe203)

En plus des nacres sur un support mica, on peut envisager les pigments multicouches basés sur des substrats synthétiques comme l'alumine, la silice, le borosilicate de calcium et de sodium ou le borosilicate de calcium et d'aluminium, et l'aluminium.

La taille du pigment utile dans le cadre de la présente invention est généralement comprise entre 10 nm et 200 µm, de préférence entre 20 nm et 80 µm, et plus préférentiellement entre 30 nm et 50 µm.

Le ou les pigments colorés ainsi que le ou les pigments ayant un indice de réfraction plus élevé que celui des pigments colorés sont chacun généralement présents dans la composition conforme à l'invention dans des quantités généralement comprises entre 0,05 et 50 % du poids total de la composition, de préférence de 0,1 à 35 %.

L'indice de réfraction des pigments peut être mesuré selon des méthodes connues de mesure de ces indices. Dans le cas des pigments organiques, l'indice de réfraction peut être mesuré selon la méthode décrite dans l'article « The refractice index of organic pigments. Its determination and significance », by A.C Cooper, B.SC., A.R.I.C. dans *Journal of the oil & colour chemists' association,* septembre 1948, N°339, Vol XXXI.

Le ou les monomères électrophiles présents dans la composition de l'invention peuvent être choisis parmi
- les dérivés benzylidene malononitrile (A), le 2-(4-chloro-benzylidene)-malononitrile (A1) le 2-cyano-3-phényl acrylate d'éthyle (B) le, 2-cyano-3-(4-chloro-phényl) acrylate d'éthyle (B1) décrits dans Sayyah, *J. Polymer Research,* **2000**, p97

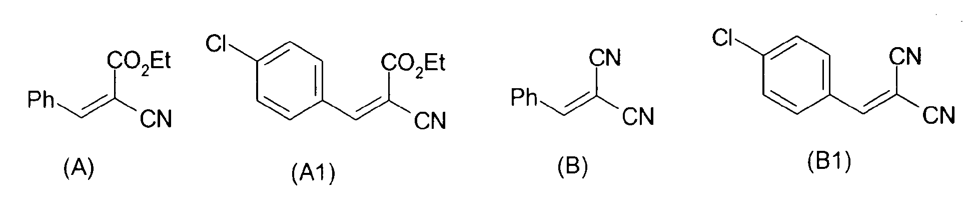

Les dérivés de méthylidenemalonates comme :
■ Le 2-méthylene-malonate de diéthyle (C) par Hopff, *Makromoleculare Chemie,* **1961**, p95, De Keyser, *J*. *Pharm. Sci,* **1991**, p67 et Klemarczyk, *Polymer,* **1998**, p173 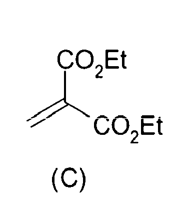
■ le 2-éthoxycarbonylméthyleneoxycarbonyl acrylate d'éthyle (D) par Breton, *Biomaterials,* **1998**, p271 et Couvreur, *Pharmaceutical Research,* **1994**, p1270.

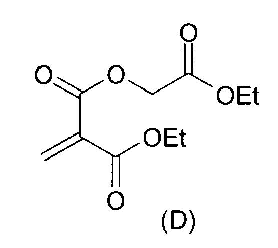

Les dérivés itaconate et itaconimide comme :
■ l'itaconate de diméthyle (E) par Bachrach, *European Polymer Journal,* **1976**, p563 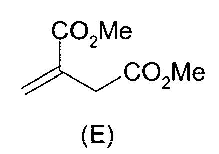
■ N-butyl itaconimide (F), N-(4-tolyl) itaconimide (G), N-(2-ethylphenyl) itaconimide (H), N-(2,6-diethylphenyl) itaconimide (I) par Wanatabe, *J.Polymer Science : Part A :Polymer chemistry,* **1994,** p2073 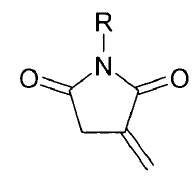 R= Bu (F), 4-tolyl (G), 2-ehylphenyl (H), 2,6-diethyphenyl (I)

Les dérivés α-(methylsulfonyl)acrylates de méthyle (K), α-(methylsulfonyl)acrylates de d'éthyle (L), α-(tert-butylsulfonyl)acrylates de méthyle (M), α-(methylsulfonyl)acrylates de tert-butyle (N) α-(tert-butylsulfonyl)acrylates de tert-butyle (O) par Gipstein, *J.Org.Chem,* **1980**, p1486 et
les dérivés 1,1-bis-(methylsulfonyl)ethylene (P), 1-acetyl-1-methyl sulfonyl ethylene (Q), α-(methylsulfonyl) vinyl sulfonate de methyle (R), α-methylsulfonylacrylonitrile (S) par Shearer, US patent US2748050.

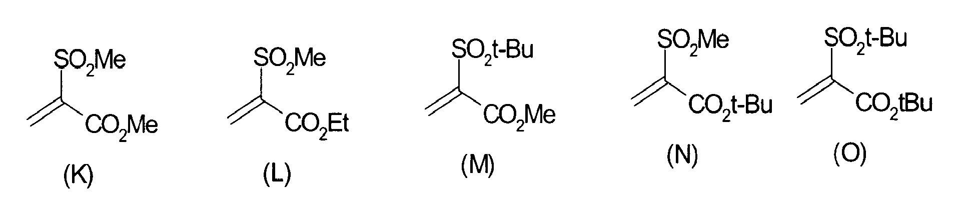

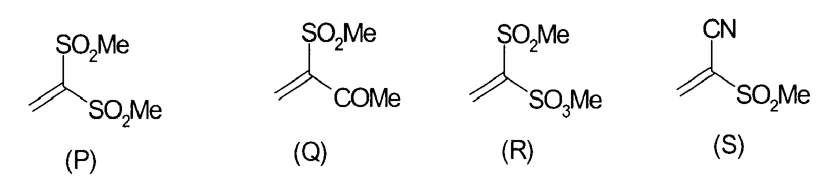

Les dérivés méthyl vinyl sulfone (T) et phényl vinyl sulfone (U) par Boor , *J. Polymer Science,* **1971**, p249

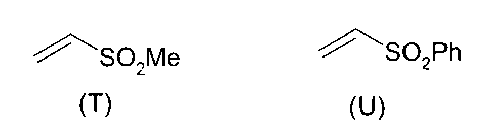

Le dérivé phényl vinyl sulfoxide (V) par Kanga, *Polymer preprints (ACS, Divison of Polymer Chemistry),* **1987**, p322

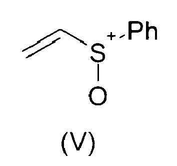

Le dérivé 3-methyl-N-(phenylsulfonyl)-1-aza-1,3-butadiene (W) par Bonner, *Polymer Bulletin,* **1992**, p517

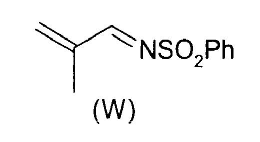

Les dérivés acrylates et acrylamides comme :
■ N-propyl-N-(3-triisopropoxysilylpropyl)acrylamide (X) et N-propyl-N-(3-triethoxysilylpropyl)acrylamide (Y) par Kobayashi, *Journal of Polymer Science, Part A: Polymer Chemistry,* **2005**, p2754. 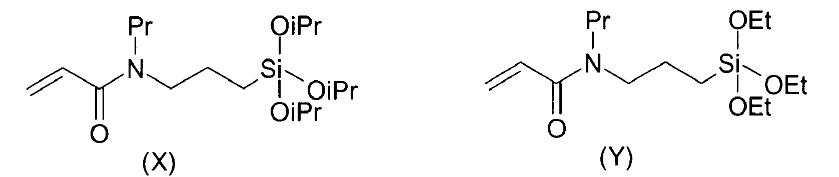
■ 2-hydroxyethyl acrylate (Z) et 2-hydroxyethyl méthacrylate (AA) par Rozenberg, *International Journal of Plastics Technology,* **2003**, p17 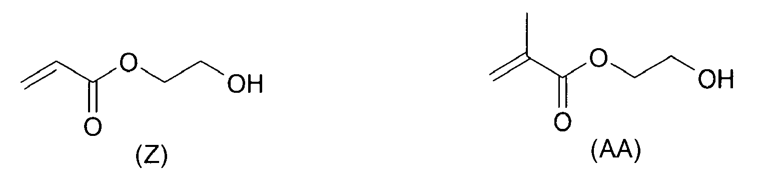
■ N-butyl acrylate (AB) par Schmitt, *Macromolecules,* **2001,** p2115
■ Tert-butyl acrylate (AC) par lshizone, *Macromolecules,* **1999**, p955.

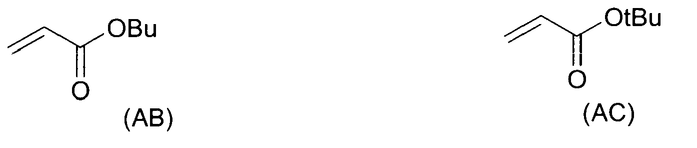

Le monomère électro-attracteur utile dans la présente invention peut être cyclique ou linéaire. Lorsqu'il est cyclique, le groupe éléctro-attracteur est de préférence exocyclique, c'est-à-dire qu'il ne fait pas partie intégrante de la structure cyclique du monomère.

Selon un mode de réalisation particulier, ces monomères présentent au moins deux groupes électro-attracteurs.

A titre d'exemple de monomères présentant au moins deux groupes électro-attracteurs, on peut citer les monomères de formule (A) : 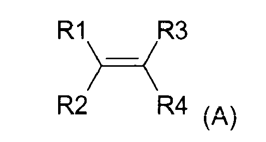 dans laquelle :
- R1 et R2 désignent chacun, indépendamment l'un de l'autre, un groupe peu ou non électro-attracteur (peu ou non inductif-attracteur) tel que :
   - un atome d'hydrogène,
   - un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR, -COOR, -COR, -SH,
   - SR, -OH, et les atomes d'halogène,
   - un résidu polyorganosiloxane modifié ou non,
   - un groupement polyoxyalkylène,
- R3 et R4 désignent chacun, indépendamment l'un de l'autre, un groupe électro-attracteur (ou inductif-attacteur) choisi de préférence parmi les groupements -N(R)3+, -S(R)2+, -SH2+, -NH3+, -NO2, -SO2R, -C≡N, -COOH, -COOR, -COSR, -CONH2, CONHR, -F, -Cl, -Br, -I, -OR, -COR, -SH, -SR, -OH, les groupes alcényle linéaires ou ramifiés, les groupes alcynyle linéaires ou ramifiés, les groupements mono- ou polyfluoroalkyle en C1-C4, les groupements aryle tels que phényle, ou les groupements aryloxy tels que phénoxyloxy,
- R désigne un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', -COOR', -COR', -SH, -SR', -OH, les atomes d'halogène, ou un résidu de polymère pouvant être obtenu par polymérisation radicalaire, par polycondensation ou par ouverture de cycle, R' désignant un groupe alkyle en C₁-C₁₀.

Par groupement électro-attracteur ou inductif-attracteur (-I), on entend tout groupement plus électronégatif que le carbone. On pourra se reporter à l'ouvrage PR Wells Prog. Phys. Org. Chem., Vol 6,111 (1968).

Par groupement peu ou non électro-attracteur, on entend tout groupement dont l'électronégativité est inférieure ou égale à celle du carbone.

Les groupements alcényle ou alcynyle ont de préférence 2 à 20 atomes de carbone, mieux encore de 2 à 10 atomes de carbone.

Comme groupe hydrocarboné saturé ou non, linéaire, ramifié ou cyclique, comportant de préférence de 1 à 20 atomes de carbone, on peut notamment citer les groupes alkyle, alcényle ou alcynyle linéaires ou ramifiés, tels que méthyle, éthyle, n-butyle, tert-butyle, iso-butyle, pentyle, hexyle, octyle, butényle ou butynyle ; les groupes cycloalkyle ou aromatiques.

Comme groupe hydrocarboné substitué, on peut citer par exemple les groupes hydroxyalkyle ou polyhalogénoalkyle.

A titre d'exemples de polyorganosiloxane non modifié, on peut notamment citer les polyalkylsiloxanes tels que les polydiméthylsiloxanes, les polyarylsiloxanes tels que les polyphénylsiloxanes, les polyarylalkylsiloxanes tels que les polyméthylphénylsiloxanes.

Parmi les polyorganosiloxanes modifiés, on peut notamment citer les polydiméthylsiloxanes à groupements polyoxyalkylène et/ou siloxy et/ou silanol et/ou amine et/ou imine et/ou fluoroalkyle.

Parmi les groupements polyoxyalkylène, on peut notamment citer les groupements polyoxyéthylène et les groupements polyoxypropylène ayant de préférence 1 à 200 motifs oxyalkylénés.

Parmi les groupements mono- ou polyfluoroalkyle, on peut notamment citer des groupements tels que -(CH2)n-(CF2)m-CF3 ou -(CH2)n-(CF2)m-CHF2 avec n=1 à 20 et m= 1 à 20.

Les substituants R1 à R4 peuvent éventuellement être substitués par un groupement ayant une activité cosmétique. Les activités cosmétiques particulièrement utilisées sont obtenues à partir de groupements à fonctions colorantes, antioxydantes, filtres UV et conditionnantes.

A titre d'exemples de groupement à fonction colorante, on peut notamment citer les groupements azoiques, quinoniques, méthiniques, cyanométhiniques et triarylméthane.

A titre d'exemples de groupement à fonction antioxydante, on peut notamment citer les groupements de type butylhydroxyanisole (BHA), butylhydroxytoluène (BHT) ou vitamine E.

A titre d'exemples de groupement à fonction filtre UV, on peut notamment citer les groupements de types benzophénones, cinnamates, benzoates, benzylidène-camphres et dibenzoylméthanes.

A titre d'exemples de groupement à fonction conditionnante, on peut notamment citer les groupements cationiques et de type esters gras.

Parmi les monomères précédemment cités, sont préférés les monomères de la famille des cyanoacrylates et leurs dérivés de formule (B) : 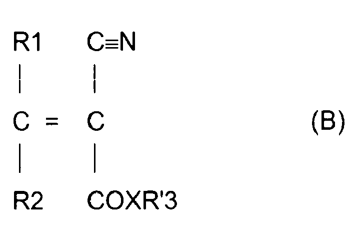 X désignant NH, S ou O,
R1 et R2 ayant les mêmes significations que précédemment, de préférence R1 et R2 représentant un atome d'hydrogène,
R'3 représentant un atome d'hydrogène ou R tel que défini à la formule (A).

De préférence, X désigne O.

A titre de composés de formule (B), on peut citer les monomères :
a) appartenant à la famille des 2-cyanoacrylates de polyfluoroalkyle tels que :
   l'ester 2,2,3,3-tétrafluoropropylique de l'acide 2-cyano-2-propénoïque de formule : 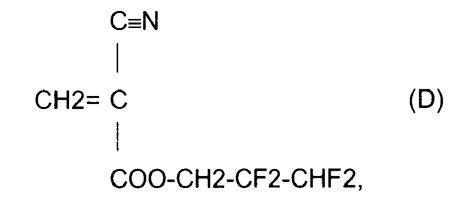
   ou encore l'ester 2,2,2-trifluoroéthylique de l'acide 2-cyano-2-propénoïque de formule : 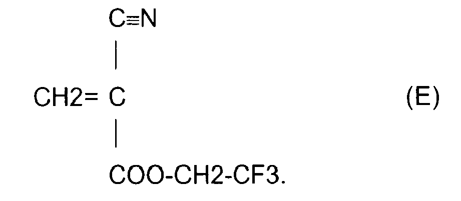
b) les 2-cyanoacrylates d'alkyle ou d'alcoxyalkyle 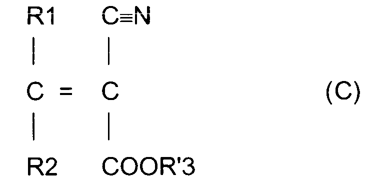 dans laquelle R'3 représente un radical alkyle en C1-C10 ou alcoxy(C1-C4) alkyle(C1-C10).

On peut citer plus particulièrement le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle et le cyanoacrylate d'iso-amyle.

Dans le cadre de l'invention, on préfère utiliser les monomères b). Selon un mode de réalisation préféré, le ou les monomères cyanoacrylates sont choisis parmi les cyanoacrylates d'alkyle en C6-C10.

Les monomères particulièrement préférés sont les cyanoacrylates d'octyle de formule F et leurs mélanges : 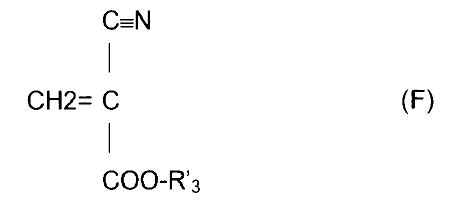 dans laquelle : R'₃ =-(CH₂)₇-CH₃,
-CH(CH₃)-(CH₂)₅-CH₃,
-CH₂-CH(C₂H₅)-(CH₂)₃-CH₃,
-(CH₂)₅-CH(CH₃)-CH₃,
-(CH₂)₄-CH(C₂H₅)-CH₃.

Les monomères utilisés conformément à l'invention peuvent être fixés de façon covalente sur des supports tels que des polymères, des oligomères ou des dendrimères. Le polymère ou l'oligomère peut être linéaire, ramifié, en peigne ou bloc. La répartition des monomères de l'invention sur la structure polymérique, oligomérique ou dendritique peut être statistique, en position terminale ou sous forme de blocs.

Dans la composition de l'invention, la quantité de monomères cyanoacrylate est comprise entre 0,1 et 80 % en poids du poids total de la composition, de préférence entre 1 et 50 %.

Dans le cadre de l'invention, les monomères électrophiles sont des monomères capables de polymériser par voie anionique en présence d'un agent nucléophile. Par polymérisation anionique, on entend le mécanisme défini dans l'ouvrage "Advanced Organic Chemistry", Third Edition de Jerry March, pages 151 à 161.

Les agents nucléophiles susceptibles d'initier la polymérisation anionique sont des systèmes connus en eux-mêmes, capables de générer un carbanion au contact d'un agent nucléophile, tels que les ions hydroxyles contenus dans l'eau. On entend par "carbanion ", les espèces chimiques définies dans " Advanced Organic Chemistry, Third Edition ", de Jerry March, page 141.

Les agents nucléophiles peuvent être peut être appliqués indépendamment de la composition de l'invention. Il peut aussi être ajouté à la composition de l'invention au moment de l'emploi.

L'agent nucléophile est un composé moléculaire, un oligomère, un dendrimère ou un polymère possédant des fonctions nucléophiles. De façon non limitative, on peut citer comme fonctions nucléophiles les fonctions : R₂N⁻, NH₂⁻ , Ph₃C⁻, R₃C⁻, PhNH⁻, pyridine, ArS⁻, R-C^{≡}C⁻, RS⁻, SH⁻, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃⁻, ClO₄⁻, H₂O, Ph représentant un groupe phényle ; Ar représentant un groupe aryle et R représentant un groupe alkyle en C1-C10.

Les monomères électrophiles peuvent être synthétisés selon les méthodes connues décrites dans la technique. En particulier, les monomères cyanoacrylates peuvent être synthétisés selon l'enseignement du US 3 527 224, US 3 591 767, US 3 667 472, US 3 995 641, US 4 035 334 et US 4 650 826.

Le milieu de coloration approprié utilisé dans la composition de l'invention est de préférence un milieu anhydre et non hygroscopique. On entend par " milieu anhydre ", un milieu contenant moins de 1 % d'eau.

Selon ce mode de réalisation, le milieu de coloration de la composition de l'invention est de préférence choisi parmi :
les alcools aromatiques tels que l'alcool benzylique ;
les alcools gras ;
les polyols modifiés ou non tels que le glycérol, le glycol, le propylène glycol, le dipropylène glycol, le butylène glycol, le butyle diglycol ;
les silicones volatiles ou non telles que la cylopentasiloxane, la cyclohexasiloxane, les polydiméthylsiloxanes modifiées ou non par des fonctions phényl et/ou siloxy et/ou silanol et/ou amine et/ou imine et/ou fluoroalkyl et/ou carboxylique et/ou betaïne et/ou ammonium quaternaire et/ou etc. ;
les huiles minérales, organiques ou végétales ;
les cires oxyéthylénées ou non, les paraffines, les alcanes et plus particulièrement les alcanes de C₅ à C₁₀ ;
les acides gras, les amides grasses, les esters gras et plus particulièrement les benzoates ou les salicylates d'alcool gras.

Selon un mode de réalisation préféré, le milieu est constitué par une silicone tels que les polydiméthylsiloxanes et les polydiméthylsiloxanes modifiées.

De préférence, les composés organiques sont choisis parmi les composés liquides à la température de 25°C et sous 105 Pa (760mm de Hg).

Le milieu de coloration de la composition de l'invention peut aussi se présenter sous forme d'une émulsion et/ou être encapsulé, les monomères électrophiles étant maintenus dans un milieu anhydre jusqu'au moment de l'utilisation. Lorsque le milieu de coloration est une émulsion, cette émulsion est par exemple constituée par une phase dispersée ou continue qui peut être constituée par de l'eau, des alcools aliphatiques en C1-C4 ou leurs mélanges et une phase organique anhydre comprenant le monomère.Dans le cas des capsules ou microcapsules, la capsule peut contenir le monomères dans un milieu anhydre et être dispersées dans un milieu anhydre tel que défini précédemment, de l'eau, des alcools aliphatiques en C1-C4 ou leurs mélanges.

On peut également introduire dans la composition de l'invention des inhibiteurs de polymérisation, et plus particulièrement des inhibiteurs de polymérisation anioniques et/ou radicalaires, ceci afin d'accroître la stabilité de la composition dans le temps. De façon non limitative, on peut citer les inhibiteurs de polymérisation suivants : le dioxyde de soufre, l'oxyde nitrique, les acides organiques tels qu'un acide sulfonique ou l'acide phosphorique, l'acide acétique, la lactone, le trifluorure de bore, l'hydroquinone et ses dérivés tels que l'hydroquinone monéthyléther, la tertiobutylhydroquinone, la benzoquinone et ses dérivés tels que la duroquinone, le catéchol et ses dérivés tels que le t-butyl catéchol et le méthoxycatéchol, l'anisole et ses dérivés tels que le méthoxyanisole ou l'hydroxyanisole, le pyrogallol et ses dérivés, le p-méthoxyphénol, l'hydroxybutyl toluène, les alkyl sulfates, les alkyl sulfites, les alkyl sulfones, les alkyl sulfoxydes, les alkyl sulfures, les mercaptans, et leurs mélanges. Les groupements alkyle désignent de préférence des groupements ayant 1 à 6 atomes de carbone.

La concentration en inhibiteur dans la composition de l'invention peut être comprise entre 10 ppm et 10 %, et plus préférentiellement entre 50 ppm et 5% en poids.

La composition de l'invention peut aussi contenir un ou plusieurs polymères ne présentant pas de réactivité sur les monomères électrophiles et capable d'augmenter la viscosité de la composition. L'augmentation de la viscosité permet de réduire la vitesse de polymérisation des monomères électrophile. Pour ce faire, on peut ajouter à la composition de l'invention et de façon non exhaustive le polyméthylméthacrylate (PMMA) ou encore les copolymères à base de cyanoacrylate tels qu'ils sont décrits dans le brevet US 6,224,622.

La composition de l'invention peut aussi contenir des charges. Cela comprend à titre non limitatif des particules incolores ou blanches, minérales ou de synthèse, lamellaires ou non lamellaires. Elles peuvent être présentes à raison de 0 à 48 % en poids, par rapport au poids total de la composition, de préférence 0,01 à 30 % en poids, et mieux de 0,02 % à 20 % en poids. On peut notamment citer le talc, le stéarate de zinc, le mica, le kaolin, les poudres de polyamide (Nylon@) (Orgasol de chez Atochem), les poudres de polyéthylène, les poudres de polymères de tétrafluoroéthylène (TéflonB), l'amidon, le nitrure de bore, des microsphères polymériques telles que celles de chlorure de polyvinylidène/acrylonitrile comme l'Expancel (Nobel Industrie), de copolymères d'acide acrylique (PolytrapB de la société Dow Corning) et les microbilles de résine de silicone (TospearlsO de Toshiba, par exemple), les organopolysiloxanes élastomères.

La composition de l'invention peut aussi contenir un ou plusieurs pigments classiques. Elle peut aussi contenir des poudres ou particules métalliques telles que des poudres ou particules d'aluminium, de zinc, de cuivre, etc.

La composition peut aussi contenir les actifs cosmétiques habituellement utilisés. On peut citer à titre non limitatif les agents réducteurs, les agents oxydants, les corps gras, les silicones, les agents épaississants, les adoucissants, les agents anti-mousse, les agents hydratants, les agents émollients, les agents alcalinisants, les élastomères, les plastifiants, les filtres solaires, les colorants directs ou d'oxydation, les argiles, les minéraux colloïdaux, les parfums, les peptisants, les conservateurs, les tensioactifs anioniques cationiques, amphotères, zwitterionqiues ou non-ionques, les polymères fixants ou non, les polymères conditionneurs, les protéines, les vitamines...

Ces compositions peuvent se présenter sous des formes diverses, telles que des lotions, des sprays, des mousses et être appliquées sous forme de shampooing ou d'après-shampooing.

Dans le cas des sprays, la composition de l'invention peut contenir un propulseur. Le propulseur est constitué par les gaz comprimés ou liquéfiés usuellement employés pour la préparation de compositions aérosols. On emploiera de manière préférentielle l'air, le gaz carbonique, l'azote comprimé ou encore un gaz soluble tel que le diméthyléther, les hydrocarbures halogénés (fluorés en particuliers) ou non et leurs mélanges.

Selon le procédé de l'invention, la composition de l'invention est appliquée sur les matières kératiniques, en particulier les fibres kératiniques tettes que les cheveux, en présence d'un agent nucléophile.

Selon un mode de réalisation particulier du procédé de l'invention, l'agent nucléophile capable d'initier la polymérisation du monomère cyanoacrylate peut être appliqué au préalable sur les fibres kératiniques. L'agent nucléophile peut être utilisé pur, en solution, sous forme d'une émulsion ou être encapsulé. Il peut aussi être ajouté à la composition anhydre au moment de l'emploi juste avant l'application sur les fibres kératiniques.

De préférence, cet agent nucléophile est l'eau. Cette eau peut être apportée par exemple par humidification préalable des fibres kératiniques. Elle peut aussi être ajoutée directement dans la composition avant application.

Selon un mode de réalisation particulier, il est possible de moduler la cinétique de polymérisation en humidifiant préalablement la fibre à l'aide d'une solution aqueuse dont le pH a été ajusté à l'aide d'une base, d'un acide ou d'un mélange acide/base. L'acide et/ou la base peuvent être inorganique ou organique.

Selon une variante, le procédé de l'invention peut être mise en oeuvre en plusieurs étapes : une première étape qui consiste à appliquer une composition contenant le ou les pigments sur les fibres et une seconde étape qui consiste à appliquer une composition contenant le monomère électrophile, l'agent nucléophile étant présent dans la composition contenant le pigment ou dans une composition séparée.

Selon cette variante, la composition contenant le ou les pigments est de préférence une solution aqueuse de pigments ce qui permet une humidification de la fibre et l'initiation de la polymérisation lorsque le monomère cyanoacrylate est appliqué.

Selon le procédé de l'invention, un mode de réalisation préféré consiste à appliquer soit le monomère électrophile et les pigments à partir d'une même composition soit d'appliquer dans un premier temps le pigment, puis le monomère électrophile.

Le procédé de l'invention peut comprendre des étapes additionnels intermédiaires ou finales telles que l'application d'un produit cosmétique, une étape de rinçage, une étape de séchage. Le séchage peut être effectué au casque, au sèche cheveux et/ou au fer à lisser. En particulier, l'application des compositions conformes à l'invention peut être suivie d'un rinçage.

Il est aussi possible de réaliser des applications multiples de la composition de l'invention afin d'obtenir une superposition de couches pour atteindre des propriétés spécifiques du dépôt en termes de nature chimique, résistance mécanique, épaisseur, aspect, toucher.

Afin d'améliorer entre autre l'adhésion du poly(cyanoacrylate) formé in situ, la fibre peut être prétraitée avec tous types de polymères.

Pour moduler la cinétique de polymérisation anionique, on peut également augmenter la nucléophilie de la fibre par transformation chimique des fibres kératiniques. A titre d'exemple, on peut citer la réduction des ponts di-sulfure composant en partie la kératine en thiols avant application de la composition de l'invention. De façon non exhaustive, on peut citer comme réducteurs des ponts di-sulfure composant en partie la kératine, les composés suivants: thiosulfate de sodium anhydre, métabisulfite de sodium en poudre, thiourée, sulfite d'ammonium, acide thioglycolique, acide thiolactique, thiolactate d'ammonium, mono-thioglycolate de glycérol, thioglycolate d'ammonium, thioglycérol, acide 2,5-dihydroxybenzoique, di-thioglycolate de diammonium, thioglycolate de strontium, thioglycolate de calcium, formo-sulfoxylate de zinc, thioglycolate d'isooctyle, dl-cystéine, thioglycolate de monoéthanolamine.

L'application de la composition de l'invention peut aussi être précédée d'un traitement capillaire comme une coloration directe ou d'oxydation.

Selon la présente invention, les monomères sont de préférence choisis parmi les monomères capables de polymériser sur les fibres kératiniques dans des conditions cosmétiquement acceptables. En particulier, la polymérisation du monomère s'effectue de préférence à une température inférieure ou égale à 80°C ce qui n'empêche pas de terminer l'application par un séchage au casque, un brushing ou passage au fer plat ou à friser.

L'invention a aussi pour objet un kit de coloration comprenant une première composition qui contient un pigment et une seconde composition qui contient le ou les monomères électrophiles et éventuellement une troisième composition qui contient l'agent nucléophile. Selon ce mode de réalisation, la composition contenant le ou les pigments est une composition aqueuse et la composition contenant le ou les monomères est une composition anhydre.

Selon une première variante, le kit comprend une première composition anhydre qui comprend le ou les pigments et le ou les monomères électrophiles et une deuxième composition qui comprend un agent nucléophile.

Les exemples suivants non limitatifs permettent d'illustrer l'invention sans en limiter sa portée.

### EXEMPLES

Des essais ont été réalisés en utilisant les composés suivants :
*Monomère :* 2-octyl-2-cyanoacrylate stabilisé avec 1% d'acide phosphorique connu sous le nom de RITE LOK CON895, commercialisé par la Société CHEMENCE
*Pigment* 1: Disperse Red 122 , CI 73915 commercialisé par Sun sous le nom Sunfast Magenta 122, présentant un indice de réfraction inférieur à 2,75.
*Pigments* 2 *:* Oxyde de titane non traité commercialisé par WCD sous le nom Titanium Dioxyde USP BC 2559 présentant un indice de réfraction de 2,75.
*Milieu de coloration :*
   50% mélange poly dimethylsiloxane alpha-omega dihydroxyle /cyclopenta dimethylsiloxane (14.7/85.3) commercialisé par Dow Corning sous le nom DC 1501 Fluid
   50% de cyclopentadimethylsiloxane commercialisé par Dow Corning sous le nom DC 245 Fluid

### Exemple 1 :

Une composition aqueuse est préparée avec 10% de pigment 1 et 10 % de pigment 2. 0,5g de cette solution aqueuse est appliqué sur 1g d'une mèche de cheveux naturels propres et secs de hauteur de ton égale à 4 ce qui correspond à une nuance naturelle châtain selon la classification des nuances naturelles décrite dans "Science des Traitements Capillaires" de C. ZVIAK, Ed. Masson 1988, p. 278.

La mèche est ensuite séchée au casque, puis humidifiée avec 0,5g d'eau. Sur cette mèche humidifiée, on applique 0,5g d'une composition contenant le milieu de coloration décrit ci dessus et 10% en poids de monomères cyanoacrylates.

Après 10 minutes de pose la mèche est séchée pendant 2 minutes au sèche-cheveux.

La mèche obtenue est colorée en rose et la couleur obtenue est rémanente à au moins six shampooings. De plus, la couleur obtenue est plus visible que celle obtenue à partir d'une composition contenant uniquement le pigment 1.

### Exemple 2

On prépare une composition contenant 10 % de pigment 1 et 10 % de pigment 2 dans le milieu de coloration décrit ci dessus. Le monomère cyanoacrylate est ajouté à cette composition de manière à obtenir une concentration finale de 10% en poids de monomères. 0,5g de cette composition est appliqué sur une mèche de cheveux naturels propres et secs de hauteur de ton égale à 4, humidifiée avec 0,5g d'eau.

Après 10 minutes de pose, la mèche est séchée pendant 2 minutes au sèche-cheveux. On obtient comme précédemment une mèche colorée en rose.

La coloration ainsi obtenue est très résistante aux shampooings.

### Exemples 3 à 10

Les compositions suivantes sont préparées pour la fabrication de composition de coloration.

### Composition colorée A

- Pigment Red 122,
   commercialisé sous le nom Sunfast Magenta 122228-2410 par SUN (indice de réfraction inférieur à 2.75) 46g
- Titane dioxyde USP BC 2559 de Kronos International
   (indice de réfraction inférieur de 2.75) 46g
- Dispersant polyhydroxystearic acid commercialisé sous le nom de Octacare DSP OL 300 par AVECIA 8g

### Composition colorée L

- Pigment organique fluorescent rouge-orange commercialisé sous le nom Sunbrite SG2516 Red Orange, par la société Sun
   (indice de réfraction inférieur de 1.64) 46g
- Oxyde de fer jaune, commercialisé sous le nom Sunpuro Yellow
   C33-9001 par la société Sun
   (indice de réfraction inférieur de 2.9) 46g
- Dispersant polyhydroxystearic acid commercialisé sous le nom de Octacare DSP OL 300 par AVECIA 8g

### EXEMPLE 3. Monomère methylheptylcyanoacrylate

La composition suivante est réalisée :

| | |
|---|---|
| DC 1501 Fluid | 40g |
| DC 245 Fluid | 40g |
| Composition **A** | 10g |
| méthylheptylcyanoacrylate de Chemence | 10g |

**1.5g** de la composition est appliqué sur une mèche de 1 g de cheveux châtain propre et humide. Après 15 minutes de pause, la mèche est séchée au sèche-cheveux pendant 2 minutes.

La mèche est colorée et la coloration obtenue est résistante au shampooing. La coloration obtenue est plus visible que si une composition ne contenant que le pigment à faible indice de réfraction avait été appliquée.

### EXEMPLE 4. Monomère methylheptylcyanoacrylate avec acide acétique

La composition suivante **B** est réalisée :

| | |
|---|---|
| méthylheptylcyanoacrylate de Chemence | 97.5g |
| acide acétique glacial | 2.5g |

La composition suivante **C** est réalisée :

| | |
|---|---|
| DC 1501 Fluid | 40g |
| DC 245 Fluid | 40g |
| Composition **A** | 10g |
| Composition **B** | 10g |

**1.5g** de la composition **C** est appliqué sur une mèche de 1 g de cheveux châtain propre et humide. Après 15 minutes de pause, la mèche est séchée au sèche-cheveux pendant 2 minutes.

La mèche est colorée et la coloration obtenue est résistante au shampooing. La coloration obtenue est plus visible que si une composition ne contenant que le pigment à faible indice de réfraction avait été appliquée.

### EXEMPLE 5. Monomère ethylhexylcyanoacrylate

La composition suivante est réalisée :

| | |
|---|---|
| DC 1501 Fluid | 40g |
| DC 245 Fluid | 40g |
| Composition **A** | 10g |
| Ethylhexylcyanoacrylate 060 de Tong Shen | 10g |

**1.5g** de la composition est appliqué sur une mèche de 1 g de cheveux châtain propre et humide. Après 15 minutes de pause, la mèche est séchée au sèche-cheveux pendant 2 minutes.

La mèche est colorée et la coloration obtenue est résistante au shampooing. La coloration obtenue est plus visible que si une composition ne contenant que le pigment à faible indice de réfraction avait été appliquée.

### EXEMPLE 6. Monomère butyl cyanoacrylate avec acide acétique

La composition suivante **D** est réalisée :

| | |
|---|---|
| Butylcyanoacrylate B-60 de Tong Shen | 90g |
| acide acétique glacial | 10g |

La composition suivante **E** est réalisée :

| | |
|---|---|
| DC 1501 Fluid | 40g |
| DC 245 Fluid | 40g |
| Composition **A** | 10g |
| Composition **D** | 10g |

**1.5g** de la composition **E** est appliqué sur une mèche de 1 g de cheveux châtain propre et humide. Après 15 minutes de pause, la mèche est séchée au sèche-cheveux pendant 2 minutes.

La mèche est colorée et la coloration obtenue est résistante au shampooing. La coloration obtenue est plus visible que si une composition ne contenant que le pigment à faible indice de réfraction avait été appliquée.

### EXEMPLE 7. Monomère éthoxyéthylcyanoacrylate avec acide acétique

La composition suivante **F** est réalisée :

| | |
|---|---|
| DC 1501 Fluid | 40g |
| DC 245 Fluid | 35g |
| Composition **A** | 10g |
| Acide acétique glacial | 5g |
| Ethoxyethylcyanoacrylate EO 460 de Tong Shen | 10g |

**1.5g** de la composition **F** est appliqué sur une mèche de 1 g de cheveux châtain propre et humide. Après 15 minutes de pause, la mèche est séchée au sèche-cheveux pendant 2 minutes.

La mèche est colorée et la coloration obtenue est résistante au shampooing. La coloration obtenue est plus visible que si une composition ne contenant que le pigment à faible indice de réfraction avait été appliquée.

### EXEMPLE 8. Mélange de monomères methylheptylcyanoacrylate et ethylhexylcyanoacrylate

La composition suivante **H** est réalisée :

| | |
|---|---|
| méthylheptylcyanoacrylate de Chemence | 90g |
| ethylhexylcyanoacrylate O-60 de Tong Shen | 10g |

La composition suivante **I** est réalisée :

| | |
|---|---|
| DC 1501 Fluid | 40g |
| DC 245 Fluid | 40g |
| Composition **A** | 10g |
| Composition **H** | 10g |

**1.5g** de la composition **I** est appliqué sur une mèche de 1g de cheveux châtain propre et humide. Après 15 minutes de pause, la mèche est séchée au sèche-cheveux pendant 2 minutes.

La mèche est colorée et la coloration obtenue est résistante au shampooing. La coloration obtenue est plus visible que si une composition ne contenant que le pigment à faible indice de réfraction avait été appliquée.

### EXEMPLE 9. Mélange de monomères methylheptylcyanoacrylate et butylcyanoacrylate

La composition suivante **J** est réalisée :

| | |
|---|---|
| méthylheptylcyanoacrylate de Chemence | 70g |
| butylcyanoacrylate B-60 de Tong Shen | 30g |

La composition suivante **K** est réalisée :

| | |
|---|---|
| DC 1501 Fluid | 40g |
| DC 245 Fluid | 40g |
| Composition **A** | 10g |
| Composition **J** | 10g |

**1.5g** de la composition **K** est appliqué sur une mèche de 1 g de cheveux châtain propre et humide. Après 15 minutes de pause, la mèche est séchée au sèche-cheveux pendant 2 minutes.

La mèche est colorée et la coloration obtenue est résistante au shampooing. La coloration obtenue est plus visible que si une composition ne contenant que le pigment à faible indice de réfraction avait été appliquée.

### EXEMPLE 10. Oxyde de fer + pigment organique

La composition suivante est réalisée :

| | |
|---|---|
| DC 1501 Fluid | 40g |
| DC 245 Fluid | 40g |
| Composition **L** | 10g |
| méthylheptylcyanoacrylate de Chemence | 10g |

**0.8g** de la composition est appliqué sur une mèche de 1 g de cheveux châtain propre et humide. Après 15 minutes de pause, la mèche est séchée au sèche-cheveux pendant 2 minutes.

La mèche est colorée et la coloration obtenue est résistante au shampooing. La coloration obtenue est plus visible que si une composition ne contenant que le pigment à faible indice de réfraction avait été appliquée.

## Revendications

1. Composition de coloration comprenant, dans un milieu de coloration approprié, au moins un monomère électrophile, au moins un pigment coloré et au moins un pigment d'un indice de réfraction supérieur au pigment coloré.

2. Composition selon la revendication 1 dans laquelle le ou les monomères électrophile sont des monomères de formule (A) : 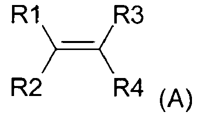 dans laquelle :
• R1 et R2 désignent chacun, indépendamment l'un de l'autre, un groupe peu ou non électro-attracteur, et
• R3 et R4 désignent chacun, indépendamment l'un de l'autre, un groupe électro-attracteur.

3. Composition selon la revendication 2 dans laquelle les monomères de formule (A) sont tels que R1 et R2, indépendamment représente un atome d'hydrogène ; un groupe hydrocarboné saturé ou non, linéaire, ramifié ou cyclique, comportant de préférence de 1 à 20, et contenant éventuellement un ou plusieurs atomes d'azote, d'oxygène, de soufre, et éventuellement substitué par un ou plusieurs groupements choisis parmi -OR, -COOR, -COR, -SH, -SR, -OH, et les atomes d'halogène ; un résidu polyorganosiloxane modifié ou non ; un groupement polyoxyalkylène, R désignant un groupe hydrocarboné saturé ou non, linéaire, ramifié ou cyclique, comportant de 1 à 20, et contenant éventuellement un ou plusieurs atomes d'azote, d'oxygène, de soufre, et éventuellement substitué par un ou plusieurs groupements choisis parmi -OR', -COOR', -COR', -SH, -SR', -OH, les atomes d'halogène, ou un résidu de polymère, R'désignant un groupe alkyle en C₁-C₁₀.

4. Composition selon la revendication 2 ou 3 dans laquelle les monomères de formule (A) sont tels que R3 et R4, indépendamment sont choisis parmi les groupements -N(R)3+, -S(R)2+, -SH2+, -NH3+, -NO2, -SO2R, -C≡N, -COOH, -COOR, -COSR, -CONHR, -CONH2, -F, -CI, -Br, -I, -OR, -COR, -SH, -SR, -OH, les groupes alcényle linéaires ou ramifiés, les groupes alcynyle linéaires ou ramifiés, les groupements mono- ou polyfluoroalkyle en C1-C4, les groupements aryle ou les groupements aryloxy , R désignant un groupe hydrocarboné saturé ou non, linéaire, ramifié ou cyclique, comportant de 1 à 20, et contenant éventuellement un ou plusieurs atomes d'azote, d'oxygène, de soufre, et éventuellement substitué par un ou plusieurs groupements choisis parmi -OR', -COOR', -COR', -SH, -SR', -OH, , les atomes d'halogène, ou un résidu de polymère, R' étant un radical alkyle en C1-C10.

5. Composition selon la revendication 1 ou 2 dans laquelle les monomères sont des monomères cyanoacrylates de formule : 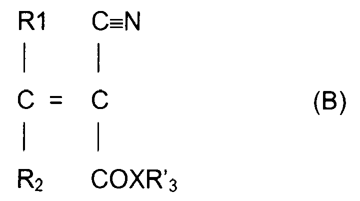 X désignant NH, S ou O,
R'₃ étant choisi parmi un atome d'hydrogène ou R,
R, R1 et R2 étant tel que défini à la revendication 3.

6. Composition selon l'une quelconque des revendications 2 à 5 dans laquelle le monomère électrophile est tel que R1 et R2 représentent un atome d'hydrogène :

7. Composition selon l'une quelconque des revendications 1 à 6 dans laquelle les monomères cyanoacrylates correspondent à la formule 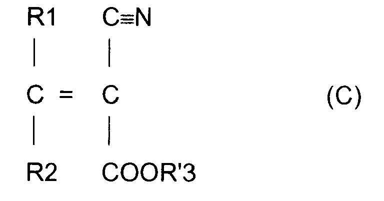 dans laquelle R'3 représente un radical alkyle en C1-C10 ou alcoxy(C1-C4)alkyle(C1-C10) et R1 et R2 sont tels que définis précédemment.

8. Composition selon la revendication 6 dans laquelle R'3 est un radical alkyle comprenant de 6 à 10 atomes de carbone.

9. Composition selon la revendication 7, dans laquelle R1 et R2 représentent l'hydrogène.

10. Composition selon l'une quelconque des revendications précédentes, dans laquelle le monomère électrophile est un cyanoacrylate d'alkyle de formule 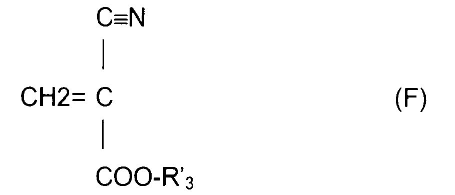 dans laquelle : R'₃ =-(CH₂)₇-CH₃,
-CH(CH₃)-(CH₂)₅-CH₃,
-CH₂-CH(C₂H₅)-(CH₂)₃-CH₃,
-(CH₂)₅-CH(CH₃)-CH₃,
-(CH₂)₄-CH(C₂H₅)-CH₃.

11. Composition selon l'une quelconque des revendications précédentes dans laquelle la quantité de monomères électrophile est comprise entre 0,1 et 80 % en poids du poids total de la composition.

12. Composition selon l'une quelconque des revendications précédentes, dans laquelle le ou les pigments sont sous forme de poudre ou de pâte pigmentaire.

13. Composition selon la revendication 12 dans laquelle le ou les pigments colorés sont des pigments organiques.

14. Composition selon la revendication 13 dans laquelle les pigments organiques sont choisis parmi les pigments de type nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

15. Composition selon la revendication 12 dans laquelle le ou les pigments colorés sont un pigment composite composé de particules comportant un noyau inorganique, au moins un liant assurant la fixation des pigments organiques sur le noyau, et au moins un pigment organique recouvrant au moins partiellement le noyau.

16. Composition selon la revendication 12 dans laquelle le ou les pigments colorés sont des laques constituées d'un substrat inorganique choisi parmi l'alumine, la silice, le borosilicate de calcium et de sodium, le borosilicate de calcium et d'aluminium, et l'aluminium sur lequel est adsorbé un colorant organique.

17. Composition selon la revendication 12 dans laquelle le pigment coloré est un pigment à effets spéciaux choisi parmi les pigments nacrés, les pigments à effets interférentiels non fixés sur un substrat, les pigments fluorescents, les pigments phosphorescents, les pigments photochromiques, les pigments thermochromiques.

18. Composition selon la revendication 12, dans laquelle le ou les pigments colorés sont des pigments nacrés choisis parmi le mica recouvert de titane et d'oxydes de fer ou d'oxyde de chrome, le mica recouvert de titane et d'un pigment organique, les pigments nacrés à base d'oxychlorure de bismuth.

19. Composition selon l'une quelconque des revendications précédentes dans laquelle le pigment ayant un indice de réfraction plus élevé que le pigment coloré est un pigment minéral.

20. Composition selon la revendication 19 dans laquelle le pigment minéral est choisi parmi les oxydes de zirconium ou de cérium, ainsi que les oxydes de fer ou de chrome, le violet de manganèse, le bleu outremer, l'hydrate de chrome et le bleu ferrique, le dioxyde de titane.

21. Composition selon la revendication 19 dans laquelle le colorant ayant un indice de réfraction plus élevé que le pigment coloré est un pigment nacré.

22. Composition selon l'une quelconque des revendications précédentes dans laquelle le ou les pigments sont chacun présents à des concentrations comprises entre 0,05 et 40 % du poids total de la composition.

23. Composition selon l'une quelconque des revendications précédentes dans laquelle la taille du pigment est de 10 nm à 200µm.

24. Composition selon l'une quelconque des revendications précédentes anhydre

25. Composition selon l'une quelconque des revendications précédentes dans laquelle le milieu de coloration est choisi parmi les alcools aromatiques, les alcools gras, les polyols modifiés ou non , les silicones volatiles ou non, les huiles minérales, organiques ou végétales, les cires oxyéthylénées ou non, les paraffines, les alcanes, les acides gras, les amides grasses, les esters gras.

26. Composition selon l'une quelconque des revendications précédentes comprenant de plus un agent nucléophile.

27. Composition selon la revendication 26 dans laquelle l'agent nucléophile est l'eau.

28. Procédé de coloration des matières kératiniques **caractérisé par le fait que** l'on applique sur les matières kératiniques la composition telle que définie à l'une quelconque des revendications 1 à 25 en présence d'un agent nucléophile.

29. Procédé selon la revendication 28 dans lequel on applique la composition telle que définie à la revendication 26 ou 27.

30. Procédé selon la revendication 28 dans lequel une première étape qui consiste à appliquer une composition contenant le ou les pigments sur les fibres et une seconde étape qui consiste à appliquer une composition contenant le monomère électrophile, l'agent nucléophile étant présent dans la composition contenant le pigment ou dans une composition séparée.

31. Procédé selon la revendication 30 dans lequel la composition contenant le ou les pigments est une composition aqueuse de pigments et la composition qui contient le ou les monomères électrophiles est anhydre.

32. Procédé selon l'une quelconque des revendications 28 à 31 pour la coloration des fibres kératiniques.

33. Kit de coloration comprenant une première composition qui contient au moins un pigment, une seconde composition qui contient le ou les monomères électrophile tel que défini aux revendications 1 à 11 et une troisième composition qui contient un agent nucléophile.

34. Kit selon la revendication 33 dans lequel la composition contenant le ou les pigments et le ou les monomères électrophiles sont présents dans la une même composition anhydre.

35. Utilisation d'une composition telle que définie aux revendications 1 à 27 pour la coloration des matières kératiniques.

36. Utilisation selon la revendication 35 pour la coloration des fibres kératiniques.
